(19) Europäisches Patentamt
European Patent Office
Office européen des brevets

(11) Numéro de publication : **0 599 697 A1**

(12) # DEMANDE DE BREVET EUROPEEN

(21) Numéro de dépôt : **93402785.5**

(22) Date de dépôt : **17.11.93**

(51) Int. Cl.$^5$ : **C07D 491/048**, C07D 495/04, A61K 31/415, // (C07D491/048, 307:00, 209:00), (C07D495/04, 333:00, 209:00)

(30) Priorité : **24.11.92 FR 9214065**
**22.06.93 FR 9307538**

(43) Date de publication de la demande :
**01.06.94 Bulletin 94/22**

(84) Etats contractants désignés :
**AT BE CH DE DK ES FR GB GR IE IT LI LU MC NL PT SE**

(71) Demandeur : **SYNTHELABO**
**22, Avenue Galilée**
**F-92350 Le Plessis Robinson (FR)**

(72) Inventeur : **Sevrin, Mireille**
**73, rue Raymond Losserand**
**F-75014 Paris (FR)**
Inventeur : **Menin, Jacques**
**Impasse des Granges Gignat**
**F-63340 St Germain Lembron (FR)**
Inventeur : **Maloizel, Christian**
**21 rue du Plateau**
**F-92190 Meudon (FR)**
Inventeur : **Diaz Martin, Juan Antonio**
**Anastro 23, 1 -C**
**E-28033 Madrid (ES)**
Inventeur : **Martin Escudero Perez, Ulpiano**
**Albuquerque 15, 6 -D**
**E-28010 Madrid (ES)**
Inventeur : **Bedoya Zurita, Manuel**
**Capitan Haya 19B, 4**
**E-28020 Madrid (ES)**
Inventeur : **Del Sol Moreno, Gregorio**
**Candido Mateos 18,**
**Portal 1, esc. 1, 6 -A**
**E-28035 Madrid (ES)**
Inventeur : **Jimenez Bargueno, Maria Dolores**
**Isidoro Fernandez 7,2 -D**
**E-28034 Madrid (ES)**
Inventeur : **Romanach Ferrer, Magali**
**Vesubiana 22. Getafe-Sector 3**
**E-28905 Madrid (ES)**

(74) Mandataire : **Thouret-Lemaitre, Elisabeth et al**
**SYNTHELABO,**
**Service Brevets,**
**B.P. 72**
**F-92352 Le Plessis-Robinson Cédex (FR)**

(54) **Dérivés de pyrrole, leur préparation et leur application en thérapeutique.**

(57) L'invention concerne des dérivés de pyrrole de formule générale (I)

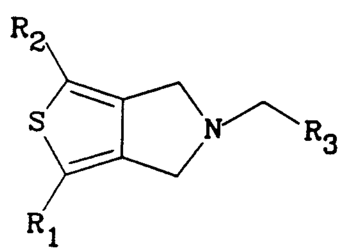

(I)

dans laquelle $R_1$ représente un atome d'hydrogène ou d'halogène, un groupe $C_{1-6}$ alkyle linéaire ou ramifié, un groupe $C_{3-6}$ alcényle, un groupe $C_{1-4}$ alcoxyméthyle, le radical benzyle, un groupe phényle éventuellement substitué par un ou plusieurs atomes d'halogène ou radicaux alkyle, un groupe $CO_2R$ dans lequel R représente un radical $C_{1-4}$ alkyle linéaire ou ramifié, le radical phényle ou le radical benzyle, ou un groupe CONR'R" dans lequel R' et R" représentent chacun, indépendamment l'un de l'autre, un atome d'hydrogène ou un radical $C_{1-4}$ alkyle linéaire ou ramifié,
$R_2$ représente un atome d'hydrogène ou d'halogène ou un groupe $C_{1-4}$ alkyle linéaire ou ramifié et $R_3$ représente un groupe 4,5-dihydro-1H-imidazol-2-yle ou 1H-imidazol-4-yle, ainsi que leurs sels d'addition à des acides pharmaceutiquement acceptables.
Application en thérapeutique.

EP 0 599 697 A1

La présente invention a pour objet des dérivés de pyrrole, leur préparation et leur application en thérapeutique.

Les composés de l'invention répondent à la formule générale (I)

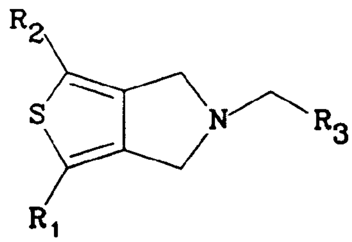

(I)

dans laquelle $R_1$ représente un atome d'hydrogène ou d'halogène, un groupe $C_{1-6}$ alkyle linéaire ou ramifié, un groupe $C_{3-6}$ alcényle, un groupe $C_{1-4}$ alcoxyméthyle, le radical benzyle, un groupe phényle éventuellement substitué par un ou plusieurs atomes d'halogène ou radicaux alkyle, un groupe $CO_2R$ dans lequel R représente un radical $C_{1-4}$ alkyle linéaire ou ramifié, le radical phényle ou le radical benzyle, ou un groupe CONR'R" dans lequel R'et R" représentent chacun, indépendamment l'un de l'autre, un atome d'hydrogène ou un radical $C_{1-4}$ alkyle linéaire ou ramifié,

$R_2$ représente un atome d'hydrogène ou d'halogène ou un groupe $C_{1-4}$ alkyle linéaire ou ramifié et $R_3$ représente un groupe 4,5-dihydro-1H-imidazol-2-yle ou 1H-imidazol-4-yle.

Les composés de l'invention forment avec les acides pharmaceutiquement acceptables des sels qui font partie de l'invention.

Les composés de formule (I) sont préparés selon le procédé représenté dans l'annexe 1, qui consiste à faire réagir un composé de formule (II)

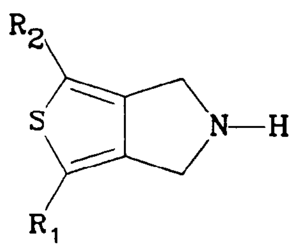

(II)

dans laquelle $R_1$ et $R_2$ ont les significations données ci-dessus, avec le 2-chlorométhyl-4,5-dihydro-1H-imidazole ou le 4-chlorométhyl-1-triphénylméthylimidazole, dans un solvant tel que le diméthylformamide, en présence de N,N-diisopropyléthylamine, dans un bain soumis aux ultrasons, pour obtenir respectivement le composé de formule (I) dans laquelle $R_3$ est le groupe 4,5-dihydro-1H-imidazol-2-yle ou un dérivé de 1-triphénylméthylimidazol-4-yle de formule (Ia) qui est ensuite déprotégé pour donner le composé de formule (I) dans laquelle $R_3$ est le groupe 1H-imidazol-4-yle.

Les composés de formule (I) dans laquelle $R_3$ est un groupe 4,5-dihydro-1H-imidazol-2-yle peuvent également être préparés selon le procédé représenté dans l'annexe 2.

Ce procédé consiste à faire réagir un composé de formule (A), dans laquelle Y représente un atome d'halogène, avec le 2-aminoacétate d'éthyle, en présence de carbonate de potassium, dans un solvant tel que le diméthylformamide ou le diméthylsulfoxyde, à la température ambiante, puis à faire réagir le dérivé de 2-(5,6-dihydro-4*H*-thiéno[3,4-*c*]pyrrol-5-yl)acétate d'éthyle obtenu avec l'éthylènediamine, en présence de triméthylaluminium, dans un solvant tel que le toluène, à la température de reflux.

Le composé de formule (I) dans laquelle $R_3$ représente un groupe 1H-imidazol-4-yle et $R_1$ et $R_2$ représentent des atomes d'hydrogène, peut également être préparé par réaction du composé de formule (Ia) dans laquelle $R_1$ et $R_2$ sont des atomes de brome, avec le formiate d'ammonium en présence de charbon palladié, suivie de la déprotection du composé de formule (Ia) dans laquelle $R_1$ et $R_2$ sont des atomes d'hydrogène, ainsi obtenu.

Les procédés de préparation des composés de formule (II) dépendent de la nature des substituants $R_1$ et $R_2$. Ces procédés sont représentés dans les annexes 3 à 6.

Les procédés représentés dans l'annexe 3 concernent la préparation des composés de formule (II) dans laquelle $R_2$ représente un atome d'hydrogène et $R_1$ représente un groupe alkyle, alcényle, benzyle ou alcoxyméthyle [composés de formule (III)] ou dans laquelle $R_2$ représente un atome d'hydrogène et $R_1$ représente un groupe alkyle ramifié (composé de formule (V) et composés analogues).

Selon l'un de ces procédés, on fait réagir un composé de formule (VIII) dans laquelle $R_4$ représente un groupe tosyle ou t-butoxycarbonyle, avec une base forte, par exemple un composé de formule $R_6Li$ dans laquelle $R_6$ représente un groupe alkyle ou dialkylamino, en particulier n-butyle ou diisopropylamino, dans un solvant tel que le tétrahydrofuranne, à une température voisine de -70°C, puis avec un halogénure de formule $R_7X$ dans laquelle X représente un atome de chlore, de brome ou d'iode et $R_7$ représente un groupe alkyle, alcényle, benzyle ou alcoxyméthyle pour obtenir un composé de formule (IV), qui est ensuite traité par l'hydrure de bis(2-méthoxyéthoxy)aluminium et de sodium, dans un solvant tel que le toluène, à la température de reflux ou par l'acide bromhydrique à une température voisine de 90°C ou par l'acide trifluoroacétique, à une température voisine de 0°C, pour donner le composé de formule (III).

Selon l'autre procédé, on fait réagir le composé de formule (VIII), dans laquelle $R_4$ représente le groupe t-butoxy-carbonyle, avec une base forte telle que $R_6Li$, dans laquelle $R_6$ représente un groupe dialkylamino, puis avec l'acétone, pour obtenir le composé de formule (VII), qui, traité par l'acide acétique, dans un solvant tel que le chloroforme, en présence de chlorure de calcium puis par le formiate d'ammonium en présence de charbon palladié, donne le composé de formule (VI) qui est ensuite traité par l'acide trifluoroacétique pour donner le composé de formule (V). L'utilisation d'une autre cétone à la place de l'acétone permet d'obtenir des composés analogues où le groupe isopropyle est remplacé par un autre groupe alkyle ramifié.

Le composé de formule (VIII) dans laquelle $R_4$ représente le groupe t-butoxycarbonyle, est obtenu par réaction d'un composé de formule (X), dans laquelle $R_5$ représente un atome de chlore ou de brome, avec le dicarbonate de di(1,1-diméthyl)éthyle puis réaction du composé de formule (IX), dans laquelle $R_4$ représente le groupe t-butoxycarbonyle, avec le formiate d'ammonium en présence de charbon palladié, dans un solvant tel que le méthanol et à une température d'environ 65°C.

Le composé de formule (VIII) dans laquelle $R_4$ représente le groupe tosyle, est obtenu par réaction du composé de formule (IX), dans laquelle $R_5$ représente un atome de chlore et $R_4$ représente le groupe tosyle, avec le formiate d'ammonium en présence de charbon palladié, dans un solvant tel que le méthanol et à une température d'environ 65°C.

Les composés de formule (X) dans laquelle $R_6$ représente un atome de chlore ou un atome de brome, sont obtenus en traitant respectivement le 1,3-dichloro-5,6-dihydro-5-tosyl-4*H*-thiéno[3,4-*c*]pyrrole ou le 5,6-dihydro-5-tosyl-4*H*-thiéno[3,4-*c*]pyrrole par l'acide bromhydrique, en présence d'acide acétique.

Les procédés représentés dans l'annexe 4 concernent la préparation des composés de formule (II) dans laquelle $R_2$ représente un atome de chlore et $R_1$ représente un groupe $CO_2R$ [composés de formule (XI)] ou dans laquelle $R_2$ représente un atome d'hydrogène et $R_1$ représente un groupe CONR'R" ou $CO_2R$ [composés de formules (XIV) et (XVI)], R, R' et R" ayant les significations indiquées ci-dessus.

Selon l'un de ces procédés, on fait réagir le composé de formule (IX), dans laquelle $R_4$ représente le groupe t-butoxycarbonyle, avec une base forte telle le t-butyllithium, dans un solvant tel le tétrahydrofuranne, puis avec le dioxyde de carbone pour obtenir le composé de formule (XIII), qui est ensuite traité par un sulfate de formule $(RO)_2SO_2$, dans laquelle R est défini comme ci-dessus, pour donner le composé de formule (XII) qui est traité par l'acide trifluoroacétique pour conduire au composé de formule (XI).

Selon l'autre procédé, on transforme le composé de formule (IX), dans laquelle $R_4$ représente le groupe tosyle, en composé de formule (VIII) selon la méthode indiquée ci-dessus, puis on traite ce composé par une base forte, telle le n-butyllithium, dans un un solvant tel que le tétrahydrofuranne, à une température voisine de -70°C, puis par le dioxyde de carbone et enfin par l'acide bromhydrique dans l'acide acétique pour obtenir le composé de formule (XVII), qui est, soit traité par le dicarbonate de di-(1,1-diméthyl)éthyle, puis par le chloroformiate d'éthyle en présence de triéthylamine et enfin par une amine de formule R'R"NH, dans laquelle R' et R" sont définis comme ci-dessus, pour donner un composé de formule (XV) qui réagit ensuite avec l'acide chlorhydrique gazeux, dans un solvant tel que l'acétate d'éthyle, à une température voisine de 20°C pour donner le composé de formule (XIV), soit traité par l'acide chlorhydrique gazeux et un alcool de formule ROH, à une température d'environ 70°C, pour donner le composé de formule (XVI), dans laquelle R est défini comme ci-dessus.

Les procédés représentés dans l'annexe 5 concernent la préparation des composés de formule (II) dans laquelle l'un des substituants $R_1$ ou $R_2$ représente un atome d'hydrogène et l'autre un atome de brome [composé de formule (XVIII)] ou dans laquelle $R_2$ représente un atome d'hydrogène ou de brome et $R_1$ représente un groupe phényle [(composés de formules (XX) et (XXII)].

Selon l'un des procédés, on traite le composé de formule (IX) par une base forte, telle le t-butyllithium, dans un solvant tel le tétrahydrofuranne, à une température voisine de -70°C, puis par l'eau, pour obtenir le composé de formule (XIX).

Selon l'autre procédé, on traite le composé de formule (IX) par l'acide phénylboronique, en présence de

palladium (0) comme catalyseur et d'une base telle que le carbonate de sodium, dans un solvant tel qu'un mélange toluène-eau, à une température voisine de 90°C, pour obtenir le composé de formule (XXIII) qui est ensuite traité par le formiate d'ammonium en présence de charbon palladié, dans un solvant tel que le méthanol à une température voisine de 65°C, pour donner le composé de formule (XXI).
Les composés de formules (XVIII), (XX) et (XXII) sont obtenus respectivement à partir des composés de formules (XIX), (XXI) et (XXIII), par réaction avec l'acide trifluoroacétique.

Les composés de formule (II) dans laquelle $R_1$ et $R_2$ représentent des atomes d'hydrogène, de chlore ou de brome ou des groupes alkyle, sont obtenus selon le schéma réactionnel donné en annexe 6.
On fait réagir le composé de formule (A), dans laquelle Y est un atome d'halogène et $R_1$ et $R_2$ sont des atomes d'hydrogène ou de chlore ou des groupes alkyle, avec un arylsulfonamide tel que le phénylsulfonamide ou le p-toluènesulfonamide, dans un solvant tel que le diméthylformamide, en présence de carbonate de potassium, à une température d'environ 100°C, puis on fait réagir le composé de formule (B) obtenu, avec l'hydrure de bis(2-méthoxyéthoxy)aluminium et de sodium, dans un solvant tel que le toluène, à la température de reflux, ou bien avec l'acide bromhydrique dans de l'acide acétique, à une température d'environ 90°C.

Les composés de formule (A) sont décrits dans la littérature ou sont préparés par des méthodes connues.

Les exemples suivants illustrent l'invention.
Les exemples 1 à 5 concernent la préparation des composés de formule (II) selon les procédés représentés dans les annexes 3 à 6.
Les analyses confirment la structure des composés.

<u>Exemple 1</u> : Bromhydrate de 1-méthyl-5,6-dihydro-4H-thiéno[3,4-c]pyrrole.

1.1 5-Tosyl-5,6-dihydro-4H-thiéno[3,4-c]pyrrole.

A une solution de 28,34 g (0,81 mole) de 1,3-dichloro-5-tosyl-5, 6-dihydro-4H-thiéno[3,4-c]pyrrole dans 800ml de méthanol, on ajoute sous atmosphère d'azote, 28 g de charbon palladié à 10% contenant 50% d'eau, puis 96 g (1,52 mole) de formiate d'ammonium. Le mélange est chauffé au reflux pendant 3 heures puis on rajoute 28 g de charbon palladié humide et on maintient le reflux pendant 24 heures. Après filtration sur terre de diatomées, on évapore à siccité et on recristallise le résidu dans du méthanol. On obtient 16,64 g d'un solide blanc.
Rendement 73%. Point de fusion : 125-127°C.

1.2 1-Méthyl-5-tosyl-5,6-dihydro-4H-thiéno[3,4-c]pyrrole.

A une solution de 3,5 g (12,5 mmoles) de 5-tosyl-5,6-dihydro-4H-thiéno[3,4-c]pyrrole dans 40 ml de tétrahydrofuranne sec, on ajoute à -70°C, 9,4 ml (15 mmoles) d'une solution de butyllithium 1,6M dans de l'hexane; après 15 mn, on ajoute 1 ml (16 mmoles) de iodométhane puis on agite à température ambiante pendant 30 mn et on verse dans 200 ml d'eau. Le précipité formé est filtré et lavé avec de l'hexane; après purification sur gel de silice à l'aide du mélange éluant acétate d'éthyle/hexane 1/9, on obtient 3,16 g d'un solide blanc.
Rendement 86%. Point de fusion : 158-160°C.

1.3 Bromhydrate de 1-méthyl-5,6-dihydro-4H-thiéno[3,4-c]pyrrole.

On chauffe à 90°C dans un bain d'eau, pendant 3 heures, un mélange de 3,1 g (10,6 mmoles) de 1-méthyl-5-tosyl-5,6-dihydro-4H-thiéno[3,4-c]pyrrole et de 3,1 g (32,9 mmoles) de phénol dans 43 ml d'une solution à 33% d'acide bromhydrique dans de l'acide acétique, placé dans un tube scellé. On filtre ensuite le mélange réactionnel et on le verse dans 150 ml d'eau puis on l'extrait avec trois fois 150 ml d'éther diéthylique. La phase aqueuse est évaporée à siccité et le résidu lavé avec de l'acétone. On obtient 0,64 g d'un solide pâteux. Rendement 27%.

<u>Exemple 2</u> : 1,3-Dichloro-5,6-dihydro-4*H*-thiéno[3,4-*c*]pyrrole.

On chauffe à 90°C dans un bain d'eau, pendant 90 mn, un mélange de 6,98 g (0,02 mole) de 1,3-dichloro-5-tosyl-5,6-dihydro-4*H*-thiéno[3,4-*c*]pyrrole et 50 ml d'une solution à 33% d'acide bromhydrique dans de l'acide acétique, placé dans un tube scellé. On refroidit ensuite le mélange réactionnel, on filtre le précipité formé, on le lave deux fois avec de l'éther diéthylique et on le sèche. On obtient 4,75 g de composé sous forme de bromhydrate (Point de fusion > 270 °C). Par traitement de ce sel avec du carbonate de sodium dans de l'eau, on obtient 3,35 g de base.
Point de fusion : 80,5-82,5°C.

Exemple 3 : Trifluoroacétate de 1-bromo-3-phényl-5,6-dihydro-4H-thiéno[3,4-c]pyrrole.

3.1 Bromhydrate de 1,3-dibromo-5,6-dihydro-4*H*-thiéno[3,4-*c*]pyrrole.

On chauffe à 90°C pendant 1 h, un mélange de 5,5 g (0,019mole) de 5-tosyl-5,6-dihydro-4*H*-thiéno[3,4-*c*]pyrrole et 60 ml d'une solution d'acide bromhydrique à 33% dans de l'acide acétique. On refroidit ensuite le mélange, on filtre le précipité formé, on le lave 2 fois avec de l'éther diéthylique et on le sèche. On obtient 4,6 g de produit.

Point de fusion : > 270°C.

3.2 1,3-Dibromo-5-[(1,1-diméthyl)éthoxy]carbonyl-5,6-dihydro-4H-thiéno[3,4-c]pyrrole.

Sur une suspension de 1 g (2,74 mmoles) de bromhydrate de 1,3-dibromo-5,6-dihydro-4H-thiéno[3,4-c]pyrrole dans 6,5 ml de dioxanne, on verse goutte à goutte et à 0°C, une solution de 0,9 g (4,12 mmoles) de dicarbonate de di(1,1-diméthyl)éthyle dans 3,5 ml de dioxanne puis 4 ml d'une solution d'hydroxyde de sodium 2M. Après 1 heure d'agitation à la température ambiante, on filtre le précipité, on le rince avec deux fois 5ml d'eau et on le sèche à 70°C. On obtient 0,95 g d'un solide blanc.

Rendement 91%. Point de fusion : 144-145°C.

3.3 1-Bromo-3-phényl-5-[(1,1-diméthyl)éthoxy]carbonyl-5,6-dihydro-4H-thiéno[3,4-c]pyrrole.

A une solution de 3,2 g (8,3 mmoles) de 1,3-dibromo-5-[(1,1-diméthyl)éthoxy]carbonyl-5,6-dihydro-4H-thiéno[3,4-c]pyrrole dans 45 ml de toluène, on ajoute 0,91 g (7,5 mmoles) d'acide phénylboronique, 0,209 g (0,38 mmol) de dibenzylidèneacétone palladium(0), 0,397 g (1,5 mmol) de triphénylphosphine et 7,5 ml (15 mmoles) d'une solution de carbonate de sodium 2M. Le mélange est chauffé au reflux pendant 5,5 heures puis refroidi. La phase organique est décantée, lavée à l'eau, séchée sur sulfate de magnésium puis évaporée à siccité. Le résidu est purifié par chromatographie sur gel de silice à l'aide du mélange éluant acétate d'éthyle/hexane 5/95. On obtient 0,98 g d'un solide blanc.

Rendement 34%. Point de fusion : 139-140°C.

3.4 Trifluoroacétate de 1-bromo-3-phényl-5,6-dihydro-4H-thiéno[3,4-c]pyrrole.

A 0,98 g (1,32 mmole) de 1-bromo-3-phényl-5-[(1,1-diméthyl)éthoxy]carbonyl-5,6-dihydro-4H-thiéno[3,4-c]pyrrole, on ajoute 1,5 ml d'acide trifluoroacétique. Après 10 minutes à la température ambiante, le solvant est évaporé à sec. On ajoute 5 ml de toluène et on évapore à sec. Le résidu est trituré avec 5 ml d'éther diéthylique, filtré et séché. On obtient 0,95 g d'un solide jaunâtre. Rendement 91%.

Exemple 4 : 1,3-Diméthyl-5,6-dihydro-4*H*-thiéno[3,4-*c*]pyrrole.

4.1 1,3-Diméthyl-5-tosyl-5,6-dihydro-4*H*-thiéno[3,4,*c*] pyrrole.

Sur une suspension de 180 g de carbonate de potassium dans 900 ml de diméthylformamide, chauffée à 100-110°C et sous agitation, on verse goutte à goutte, en 6 h, une solution de 10 g (0,048 mole) de 3,4-bis(chlorométhyl)-2,5-diméthylthiophène et 8,22 g de p-toluènesulfonamide dans 600 ml de diméthylformamide sec.

Après la fin de l'addition, on maintient le mélange à la même température pendant 30 mn, puis on le filtre et on lave le solide avec du diméthylformamide. On concentre le filtrat sous vide, on traite le résidu avec 100ml d'éthanol, puis on filtre, on lave le solide restant, avec de l'éthanol et on le sèche.

On obtient 9,39 g de produit.

Point de fusion : 163-164°C.

4.2 1,3-Diméthyl-5,6-dihydro-4*H*-thiéno[3,4-*c*]pyrrole.

Sur une suspension de 9,1 g (0,03 mole) de 1,3-diméthyl-5-tosyl-5,6-dihydro-4*H*-thiéno[3,4-*c*]pyrrole dans 42 ml de toluène sec, on verse, à la température ambiante, un mélange de 38 ml d'une solution 3,4 M d'hydrure de bis(2-méthoxyéthoxy)aluminium et de sodium dans du toluène et 10 ml de toluène sec. On chauffe ensuite le mélange au reflux pendant 2 h 30 mn puis on le refroidit à la température ambiante et on le verse dans 400 ml d'hydroxyde de sodium 1N. On lave la phase organique avec 3 fois 50 ml d'eau et on l'extrait avec une solution d'acide chlorhydrique 0,5N. Le solide, précipité par addition d'une solution de carbonate de sodium, est filtré, lavé avec de l'eau et séché.

On obtient 3,12 g de produit.

Point de fusion : 110-112°C (avec décomposition).

Exemple 5 : Trifluoroacétate de 1-[(1-méthyl)éthyl]-5,6-dihydro-4H-thiéno[3,4-c]pyrrole.

5.1 1,3-Dichloro-5-[(1,1-diméthyl)éthoxy]carbonyl-5,6-dihydro-4H-thiéno[3,4-c]pyrrole.

Sur une suspension de 50 g (182 mmoles) de bromhydrate de 1,3-dichloro-5,6-dihydro-4H-thiéno-[3,4-c]pyrrole dans 500 ml de dioxanne on verse goutte à goutte, à 0°C, une solution de 61,64 g (282 mmoles) de dicarbonate de di(1,1-diméthyl)éthyle dans 275 ml de dioxanne puis 274 ml d'une solution d'hydroxyde de sodium 2M. Après 1 heure d'agitation à la température ambiante, on filtre le précipité, on le rince avec deux fois 500 ml d'eau et on le sèche à 70°C. On obtient 51 g d'un solide blanc.
Rendement 95%. Point de fusion : 112-114°C.

5.2 5-[(1,1-Diméthyl)éthoxy]carbonyl-5,6-dihydro-4H-thiéno[3,4-c]pyrrole.

A une solution de 8,82 g (30 mmoles) de 1,3-dichloro-5-[(1,1-diméthyl)éthoxy]carbonyl-5,6-dihydro-4H-thiéno[3,4-c]pyrrole dans 75 ml de méthanol on ajoute 17,6 g de charbon palladié à 10% contenant 50% d'eau puis 41,6 g (600 mmoles) de formiate d'ammonium. On chauffe au reflux pendant 24 heures, puis on refroidit, filtre sur colonne de célite, rince avec deux fois 50 ml de dichlorométhane et évapore à sec. Le résidu est repris avec 100 ml de dichlorométhane, filtré et évaporé à siccité. On obtient 4,54 g d'un produit huileux qui cristallise lentement.
Rendement 68%. Point de fusion : 36-38°C.

5.3 1-(2-Hydroxy-2-propyl)-5-[(1,1-diméthyl)éthoxy]carbonyl-5,6-dihydro-4H-thiéno[3,4-c]pyrrole.

A une solution de 0,7 ml (5 mmoles) de N,N-diisopropylamine dans 20 ml de tétrahydrofuranne sec, on ajoute à 0°C, 3,15 ml (5 mmoles) d'une solution de butyllithium 1,6M dans de l'hexane; après 30 mn, on refroidit à -70°C et on ajoute une solution de 0,95 g (4,2 mmoles) de 5-[(1,1-diméthyl)éthoxy]carbonyl-5,6-dihydro-4H-thiéno[3,4-c]pyrrole dans 15 ml de tétrahydrofuranne; après 1 heure à -70°C, on ajoute 1,54 ml (21 mmoles) d'acétone puis on agite à température ambiante pendant une nuit. Le mélange réactionnel est versé dans 50 ml d'eau puis extrait avec trois fois 30 ml de dichlorométhane; les phases organiques sont séchées sur sulfate de sodium puis évaporées à siccité. Le résidu est chromatographié sur colonne de gel de silice à l'aide du mélange éluant acétate d'éthyle/cyclohexane 1/4. On obtient 0,425 g d'un solide blanc.
Rendement 36%. Point de fusion : 113,5°C.

5.4 1-(1-Propèn-2-yl)-5-[(1,1-diméthyl)éthoxy]carbonyl-5,6-dihydro-4H-thiéno[3,4-c]pyrrole.

A une solution de 0,81 g (2,86 mmoles) de 1-(2-hydroxy-2-propyl)-5-[(1,1-diméthyl)éthoxy]carbonyl-5,6-dihydro-4H-thiéno[3,4-c]pyrrole dans 60 ml de chloroforme, on ajoute 5 g de chlorure de calcium et 0,17 ml (3 mmoles) d'acide acétique. Le mélange est chauffé au reflux pendant 24 heures, filtré, lavé avec une solution de bicarbonate de sodium à 5% puis séché sur sulfate de sodium et évaporé à sec. On obtient 0,78 g d'un produit huileux.

5.5 1-[(1-Méthyl)éthyl]-5-[(1,1-diméthyl)éthoxy]carbonyl-5,6-dihydro-4H-thiéno[3,4-c]pyrrole.

A une solution de 0,78 g (2,9 mmoles) de 1-(1-propèn-2-yl)-5-[(1,1-diméthyl)éthoxy]carbonyl-5,6-dihydro-4H-thiéno[3,4-c]pyrrole dans 40 ml de méthanol on ajoute 3,7 g (59 mmoles) de formiate d'ammonium puis 0,9 g de charbon palladié à 10%. Le mélange réactionnel est chauffé au reflux pendant 8 heures, puis filtré sur colonne de célite et évaporé à sec. Le résidu est repris avec 50 ml d'acétate d'éthyle, lavé avec 20 ml d'eau, séché sur sulfate de sodium et évaporé à sec. Après purification sur colonne de silice à l'aide du mélange éluant acétate d'éthyle/hexane 1/4, on obtient 0,49 g d'un produit huileux. Rendement 63%.

5.6 Trifluoroacétate de 1-[(1-méthyl)éthyl]-5,6-dihydro-4H-thiéno[3,4-c]pyrrole.

On agite une solution de 0,49 g (1,8 mmole) de 1-[1-(1-méthyl)éthyl]-5-[(1,1-diméthyl)éthoxy]carbonyl-5,6-dihydro-4H-thiéno[3,4-c]pyrrole dans 0,5 ml d'acide trifluoroacétique pendant 30 mn à une température voisine de 20°C. Après évaporation du solvant, on obtient 0,51 g d'un produit huileux. Rendement quantitatif.

<u>Exemple 6</u> : Dichlorhydrate de 1,3-dichloro-5-[(4,5-dihydro-1*H*-imidazol-2-yl)méthyl]-5,6-dihydro-4*H*-thiéno[3,4-*c*]pyrrole.

On verse une solution de 2,13 g (0,011 mole) de 1,3-dichloro-5,6-dihydro-4*H*-thiéno[3,4-*c*]pyrrole et 2,5 ml de *N*,*N*-diisopropyléthylamine dans 25 ml de diméthylformamide, sur un mélange de 1,95 g (0,0125 mole) de chlorhydrate de 2-chlorométhyl-1*H*-4,5-dihydroimidazole, 2,5 ml de *N*,*N*-diisopropyléthylamine et 25 ml de diméthylformamide. On soumet le mélange aux ultrasons pendant 8 h puis on l'évapore sous vide. On dissout le résidu dans 75 ml d'eau salée et on l'extrait avec 5 fois 50 ml de chlorure de méthylène. On sèche la solution organique puis on la concentre pour obtenir un résidu qui est dissous dans 50 *ml* d'isopropanol et traité avec 14 ml d'une solution 1*N* d'acide chlorhydrique dans de l'isopropanol. On filtre les cristaux formés par refroidissement et on les lave à l'éther diéthylique. On obtient 0,5 g de produit.
Point de fusion : 206-213°C (avec décomposition).

<u>Exemple 7</u> : Chlorhydrate de 5-[(4,5-dihydro-1*H*-imidazol-2-yl)méthyl]-5,6-dihydro-4*H*-thiéno[3,4-*c*]pyrrole.

7.1 5, 6-dihydro-4*H*-thiéno[3,4-*c*]pyrrole-5-acétate d'éthyle.

Dans un ballon de 250 ml on introduit, sous argon, 13,18 g (0,0945 mole) de chlorhydrate d'aminoacétate d'éthyle, 33,8 g (0,245 mole) de carbonate de potassium et 60 ml de diméthylformamide.
On refroidit le mélange à 5°C et ajoute en 25 minutes 8,5 g (0,0315 mole) de 3,4-bis(bromométhyl)thiophène en solution dans 30 ml de diméthylformamide.
On agite ce mélange à la température ambiante pendant 67 h.
On verse le mélange dans 500 ml d'eau glacée et extrait 3 fois à l'éther. On lave 3 fois à l'eau les phases organiques, les réunit, les sèche, les filtre puis on concentre sous pression réduite. On obtient 1,57 g (0,007 mole) de produit (rendement 24 %).

7.2 Chlorhydrate de 5-[(4,5-dihydro-1*H*-imidazol-2-yl)méthyl]-5,6-dihydro-4*H*-thiéno[3,4-*c*]pyrrole.
Dans un ballon tricol de 150 ml on introduit, sous argon, 17 ml de toluène et 8,9 ml (0,0213 mole) d'une solution de triméthylaluminium à 25 % dans de l'hexane. On refroidit le mélange à l'aide de glace et on ajoute goutte à goutte 1,28 g (0,0213 mole) d'éthylènediamine en solution dans 5 ml de toluène.
On chauffe le mélange réactionnel à 60°C et on introduit 1,5 g (0,0071 mole) de 5,6-dihydro-4*H*-thiéno[3,4-*c*]pyrrole-5-acétate d'éthyle en solution dans 17 ml de toluène.
On porte le mélange à la température du reflux, distille 15 ml de solvant et maintient l'ébullition pendant 1 h 30.
Après refroidissement, on amène la température du mélange à -10°C, ajoute 10 ml d'eau, 40 ml de dichlorométhane et 20 ml d'acétate d'éthyle et agite pendant 30 minutes.
On verse la suspension dans 100 ml d'acétate d'éthyle, lave à l'eau, sèche et évapore sous pression réduite. On obtient un produit solide (0,9 g) que l'on transforme en chlorhydrate à l'aide d'une solution d'acide chlorhydrique 0,1 N dans l'isopropanol. On recristallise dans un mélange d'isopropanol et d'acétate d'éthyle (4/1).
On obtient 0,5 g (0,002 mole) d'un solide blanc (rendement: 28 %). Point de fusion : 212-215°C.

Exemple 8 : 1,3-Dibromo-5-[(4,5-dihydro-1*H*-imidazol-2-yl)méthyl]-5,6-dihydro-4*H*-thiéno[3,4-*c*]pyrrole.

On verse une solution de 1,82 g (0,005 mole) de bromhydrate de 1,3-dibromo-5,6-dihydro-4*H*-thiéno[3,4-*c*]pyrrole et 2,5 ml de *N,N*-diisopropyléthylamine dans 25 ml de diméthylformamide, sur un mélange de 1,15 g (0,0073 mole) de chlorhydrate de 2-chlorométhyl-1H-4,5-dihydroimidazole, 1ml de *N,N*-diisopropyléthylamine et 25 ml de diméthylformamide. On soumet le mélange réactionnel aux ultrasons pendant 12 h, puis on l'évapore sous vide. On dissout le résidu dans 75 ml d'eau salée puis on extrait avec 5 fois 30 ml de chlorure de méthylène. La solution organique est séchée et concentrée pour donner un résidu qui est dissous dans 50 ml d'eau. Le précipité formé par addition de soude 5N, est lavé à l'eau.
On obtient 1,3 g de composé.
Point de fusion : 175°C (avec décomposition).

Exemple 9 : Dichlorhydrate de 1-chloro-5-[(4,5-dihydro-1*H*-imidazol-2-yl)méthyl]-5,6-dihydro-4*H*-thiéno[3,4-*c*]pyrrole.

9.1 2-Chloro-3,4-bis(hydroxyméthyl)thiophène.
On verse 20 g (0,095 mole) de 2,5-dichloro-3,4-bis(hydroxyméthyl)thiophène sur une suspension de 20 g de charbon palladié à 10% dans 500 ml de méthanol, dans laquelle on a dissous 20 g (0,35 mole) de potasse. On agite le mélange et on le chauffe au reflux tout en faisant passer un courant d'hydrogène. Au bout de 24 h, on filtre le mélange, on concentre le filtrat, on verse le résidu dans 50 ml d'eau froide et on extrait avec 2 fois 100 ml puis 6 fois 50 ml d'éther diéthylique. On sèche les phases éthérées sur sulfate de magnésium et on les concentre. On obtient 8,49 g de produit.
Point de fusion : 68-69°C.

9.2 2-chloro-3,4-bis(bromométhyl)thiophène.
On verse en 30 mn, une solution de 20 ml de tribromure de phosphore dans 75 ml de tetrachlorure de carbone sur un mélange de 8,29 g (0,0464 mole) de 2-chloro-3,4-bis(hydroxyméthyl)thiophène et 300 ml de tetrachlorure de carbone, refroidi dans un bain d'eau. On agite ensuite le mélange pendant 1 h, à la température ambiante, et on ajoute 175 ml d'eau froide. On sépare la phase organique, on la lave avec 50 ml de bicarbonate de sodium à 10% et 100 ml d'eau et on la sèche sur sulfate de magnésium. On concentre la solution pour obtenir 12,94 g d'un produit huileux qui est utilisé tel quel à l'étape suivante.

9.3 2-(1-chloro-5, 6-dihydro-4*H*-thiéno[3,4-*c*]pyrrol-5-yl)acétate d'éthyle.
On verse goutte à goutte, une solution de 12,58 g (0,041 mole) de 2-chloro-3,4-bis(bromométhyl)thiophène dans 40 ml de diméthylformamide sur un mélange de 17,77 g de chlorhydrate de 2-aminoacétate d'éthyle, 45,5 g de carbonate de potassium finement pulvérisé et 80 ml de diméthylformamide, refroidi à

5°C. On agite le mélange à la température ambiante pendant 2 h puis on le verse sur 400 g d'un mélange eau-glace et on agite encore pendant 15 mn. On extrait le mélange avec 5 fois 100 ml d'éther diéthylique, on lave la phase éthérée avec 2 fois 100 ml d'eau salée, on la sèche sur sulfate de magnésium et on la concentre. On obtient 9,49 g d'un produit huileux.

9.4 Dichlorhydrate de 1-chloro-5-[(4,5-dihydro-1*H*-imidazol-2-yl)méthyl]-5,6-dihydro-4*H*-thiéno[3,4-*c*]pyrrole.

On verse goutte à goutte, une solution de 6,85 g (0,114 mole) d'éthylènediamine dans 30 ml de toluène, sur un mélange de 90 ml de toluène sec et 57 ml (0,114 mole) de triméthylaluminium en solution 2M dans du toluène refroidi à 0°C. On chauffe ensuite le mélange à 60°C dans un bain d'huile,puis on ajoute une solution de 9,38 g (0,038 mole) de 2-(1-chloro-5,6-dihydro-4*H*-thiéno{3,4-*c*]pyrrol-5-yl)acétate d'éthyle dans 90 ml de toluène. On chauffe le mélange au reflux pendant 1 h 30 mn, puis on le refroidit à -10°C et on hydrolyse avec 60 ml d'eau en maintenant la température entre -10°C et -5°C. On ajoute 250 ml de chlorure de méthylène et 150 ml d'acétate d'éthyle, on élimine les sels minéraux par filtration, on sépare la phase organique, on la lave avec 3 fois 250 ml d'eau, on la sèche et on la concentre sous vide. On obtient 4,41 g de produit qui est dissous dans 75 ml d'isopropanol et traité avec 40 ml d'une solution 1M d'acide chlorhydrique dans de l'isopropanol. On concentre la solution et on recristallise le produit dans 45 ml d'éthanol. On obtient 4,50 g de produit.

Point de fusion : 217-219°C (avec décomposition).

Exemple 10 : Dichlorhydrate de 1-éthoxycarbonyl-5-[(4,5-dihydro-1*H*-imidazol-2-yl)méthyl]-5,6-dihydro-4*H*-thiéno[3,4-*c*]pyrrole.

10.1 1-carboxy-5-tosyl-5,6-dihydro-4H-thiéno[3 4-c]pyrrole.

A une solution de 3,5 g (12,5 mmoles) de 5-tosyl-5,6-dihydro-4H-thiéno[3,4-c]pyrrole dans 40 ml de tétrahydrofuranne sec on ajoute, sous argon et à -70°C, 9,4 ml (15 mmoles) d'une solution de butyllithium 1,5 M dans de l'hexane. Après 10 minutes, le mélange est versé sur 200 g d'anhydride carbonique solide puis évaporé à sec. On ajoute 50 ml d'eau et la suspension obtenue est filtrée puis versée sur de l'acide chlorhydrique 1 M. On obtient un précipité qui est filtré et séché pour fournir 2,85 g d'un solide crème.

Rendement 70%. Point de fusion : 234-236°C (avec décomposition).

10.2 Bromhydrate de 1-carboxy-5,6-dihydro-4H-thiéno[3,4-c]pyrrole.

On chauffe à 60°C dans un bain d'eau, pendant 15 mn, un mélange de 0,5 g (1,54 mmole) de 1-carboxy-5-tosyl-5,6-dihydro-4H-thiéno[3,4-c]pyrrole et 5 ml d'une solution à 33% d'acide bromhydrique dans de l'acide acétique, placé dans un tube scellé. On refroidit ensuite le mélange réactionnel, on filtre le précipité formé, on le lave deux fois avec de l'éther diéthylique et on le sèche. On obtient 0,32 g de produit sous forme de bromhydrate.

Rendement 83%. Point de fusion : 281,3-281,7°C.

10.3 Chlorhydrate de 1-éthoxycarbonyl-5,6-dihydro-4H-thiéno[3,4-c]pyrrole.

On fait passer un courant d'acide chlorhydrique dans une suspension de 3,3 g (13,2 mmoles) de bromhydrate de 1-carboxy-5,6-dihydro-4H-thiéno[3,4-c]pyrrole dans 75 ml d'éthanol, chauffée au reflux pendant 4 h. Le solvant est ensuite évaporé et le résidu trituré avec deux fois 20 ml d'éther diéthylique. On obtient 2,46 g d'un solide beige.

Rendement 80%. Point de fusion : 163°C (avec décomposition).

10.4 Dichlorhydrate de 1-éthoxycarbonyl-5-[(4,5-dihydro-1*H*-imidazol-2-yl)méthyl]-5,6-dihydro-4*H*-thiéno[3,4-*c*]pyrrole.

On verse une solution de 0,65 g de chlorhydrate de 1-éthoxycarbonyl-5,6-dihydro-4*H*-thiéno[3,4-*c*]pyrrole et 1,47 ml de N,N-diisopropyléthylamine dans 15 ml de diméthylformamide sur un mélange de 0,75 g de chlorhydrate de 2-chlorométhyl-1*H*-4,5-dihydroimidazole, 0,5 ml de N,N-diisopropyléthylamine et 15 ml de diméthylformamide. On soumet le mélange aux ultrasons pendant 4 h, puis on l'évapore sous vide. On dissout le résidu dans 3 ml d'éthanol et on chromatographie sur colonne de silice avec de l'éthanol comme éluant. Après évaporation du solvant, on traite le résidu avec 5 ml d'isopropanol saturé en acide chlorhydrique.

On obtient 0,182 g de produit.

Point de fusion : 213-215°C (avec décomposition).

Exemple 11 : Dichlorhydrate de 1-éthyl-5-[(4,5-dihydro-1*H*-imidazol-2-yl)méthyl]-5,6-dihydro-4*H*-thiéno[3,4-*c*]pyrrole.

11.1 1-Ethyl-5-tosyl-5,6-dihydro-4*H*-thiéno[3,4-*c*]pyrrole

A une solution de 1,4 g de 5-tosyl-5,6-dihydro-4*H*-thiéno[3,4-*c*]pyrrole dans 15 ml de tétrahydrofuranne sec, on ajoute, à -70°C, 3,75 ml d'une solution 1,6 M de n-butyllithium dans de l'hexane. On ajoute ensuite 0,51 ml de iodoéthane et on agite le mélange à la température ambiante pendant 2 h, puis on verse la solution dans 100 ml d'eau glacée. On filtre le solide formé, on le lave avec de l'hexane et on le recristallise dans de l'éthanol. On obtient 0,16 g de produit.

Point de fusion : 117-120°C.

11.2 Chlorhydrate de 1-éthyl-5,6-dihydro-4*H*-thiéno[3,4-*c*]pyrrole

Sur une solution de 4,8 g de 1-éthyl-5-tosyl-5,6-dihydro-4*H*-thiéno[3,4-*c*]pyrrole dans 50 ml de toluène sec, on verse à la température ambiante, 20 ml d'une solution 3,4 M d'hydrure de bis(2-méthoxyéthoxy)aluminium et de sodium dans du toluène. On chauffe le mélange au reflux pendant 26 h, puis on le refroidit à la température ambiante et on le verse dans 300 ml d'hydroxyde de sodium 1N. On lave la phase organique avec 3 fois 10 ml d'eau puis on l'évapore à sec. On dissout le résidu dans 5 ml d'isopropanol saturé d'acide chlorhydrique.

On filtre ensuite les cristaux formés, on les lave avec de l'acétone et on les sèche. On obtient 0,3 g de produit.

Point de fusion : 149-151°C.

11.3 Dichlorhydrate de 1-éthyl-5-[(4,5-dihydro-1*H*-imidazol-2-yl)méthyl]-5,6-dihydro-4*H*-thiéno[3,4-*c*]pyrrole

Dans un ballon de 25 ml, on introduit 0,53 g de chlorhydrate de 1-éthyl-5,6-dihydro-4*H*-thiéno[3,4-*c*]pyrrole, 1,47 ml de N,N-diisopropyléthylamine et 15 ml de diméthylformamide puis on ajoute une solution de 0,75 g de chlorhydrate de 2-chlorométhyl-1*H*-4,5-dihydroimidazole et 0,5 ml de N,N-diisopropyléthylamine dans 15 ml de diméthylformamide. On soumet le mélange réactionnel aux ultrasons pendant 8 h, puis on évapore le solvant et on purifie le résidu par chromatographie sur gel de silice avec de l'éthanol comme éluant. On recristallise le produit dans 5 ml d'isopropanol saturé en acide chlorhydrique. On obtient 0,233 g de produit.

Point de fusion : 207-208°C (avec décomposition).

<u>Exemple 12</u> : Dichlorhydrate de 1,3-dibromo-5-[(1*H*-imidazol-4-yl)méthyl]-5,6-dihydro-4*H*-thiéno[3,4-*c*]pyrrole.

12.1 1,3-Dibromo-5-[(1-triphénylméthyl-1*H*-imidazol-4-yl)méthyl]-5,6-dihydro-4*H*-thiéno[3,4-*c*]pyrrole.

Dans un ballon de 50 ml, on introduit 1,5 g (4,1 mmoles) de bromhydrate de 1,3-dibromo-5,6-dihydro-4*H*-thiéno-[3,4-*c*]pyrrole, 2,12 g (5,9 mmoles) de 1-triphénylméthyl-4-chloro-méthylimidazole, 30 ml de diméthylformamide et 1,54 ml (9 mmoles) de N,N-diisopropyléthylamine. On soumet le mélange réactionnel aux ultrasons pendant 2 heures puis on le verse sur de la glace. Le précipité obtenu est filtré, lavé à l'eau, repris avec 50 ml d'acétate d'éthyle, séché sur sulfate de sodium, filtré et évaporé à sec. Le résidu est purifié par chromatographie sur gel de silice à l'aide du mélange éluant acétate d'éthyle/hexane 1/1. On obtient 0,8 g d'un solide blanc. Rendement 32%.

12.2 Dichlorhydrate de 1,3-dibromo-5-[(1*H*-imidazol-4-yl)méthyl]-5,6-dihydro-4*H*-thiéno[3,4-*c*]pyrrole.

On chauffe au reflux pendant 1 heure, une suspension de 0,7 g (1,15 mmole) de 1,3-dibromo-5-[(1-triphénylméthyl-1*H*-imidazol-4-yl)méthyl]-5,6-dihydro-4*H*-thiéno[3,4-*c*]pyrrole dans 14 ml d'acide chlorhydrique 2M. Le précipité formé est filtré et lavé à l'eau. Les phases aqueuses réunies sont lavées avec de l'acétate d'éthyle puis évaporées à siccité pour fournir 0,45 g d'un solide marron qui est cristallisé dans de l'isopropanol. On obtient 0,082 g d'un solide crème.

Rendement 17%. Point de fusion : 221°C (avec décomposition).

<u>Exemple 13</u> : Dichlorhydrate de 5-[(1*H*-imidazol-4-yl)méthyl]-5,6-dihydro-4*H*-thiéno[3,4-*c*]pyrrole.

13.1 5-[(1-Triphénylméthyl-1*H*-imidazol-4-yl)méthyl]-5,6-dihydro-4*H*-thiéno[3,4-*c*]pyrrole.

Dans un ballon de 100 ml, on introduit 0,88 g (1,45 mmole) de 1,3-dibromo-5-[(1-triphénylméthyl-1*H*-imidazol-4-yl)méthyl]-5,6-dihydro-4*H*-thiéno[3,4-*c*]pyrrole et 70 ml de méthanol. A la suspension obtenue, on ajoute 1,83 g (29 mmoles) de formiate d'ammonium et 0,6 g de charbon palladié à 10%. Le mélange est chauffé au reflux pendant 8 heures puis refroidi et filtré sur célite. Le résidu est lavé avec deux fois 10 ml de dichlorométhane et les filtrats sont évaporés à siccité.

On obtient 0,34 g d'un solide qui est purifié par chromatographie sur gel de silice à l'aide du mélange éluant dichlorométhane/méthanol 29/1. On obtient 0,14 g de composé avec un rendement de 23%.

13.2 Dichlorhydrate de 5-[(1*H*-imidazol-4-yl)méthyl]-5,6-dihydro-4*H*-thiéno[3,4-*c*]pyrrole.

On dissout 0,14 g (0,3 mmole) de 5-[(1-triphénylméthyl-1*H*-imidazol-4-yl)méthyl]-5,6-dihydro-4*H*-thiéno[3,4-*c*]pyrrole dans 1 ml d'éthanol puis on ajoute 1,5 ml d'acide chlorhydrique 2M et on chauffe au reflux pendant 1 heure. Le précipité formé est filtré et le filtrat est lavé avec deux fois 1 ml d'acétate d'éthyle puis évaporé à sec. Le résidu est dissous dans 2 ml de méthanol et décoloré par du charbon actif puis recristallisé dans de l'isopropanol. On obtient 0,075 g de solide blanc crème avec un rendement de 86%. Point de fusion : 260°C.

Les composés de l'invention sont rassemblés dans les tableaux I et II avec leurs caractéristiques physiques.

Tableau I

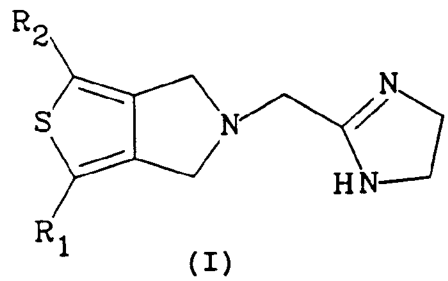

(I)

| Composé | $R_1$ | $R_2$ | base ou sel | F (°C) |
|---|---|---|---|---|
| 1 | $CH_3$ | H | dichlorhydrate | 208-210 (d) |
| 2 | $CH_2CH_3$ | H | dichlorhydrate | 207-208 (d) |
| 3 | $(CH_2)_2CH_3$ | H | dichlorhydrate | 186-189 (d) |
| 4 | $(CH_2)_3CH_3$ | H | dichlorhydrate | 196-198 (d) |
| 5 | $CH(CH_3)_2$ | H | dichlorhydrate | 213 (d) |
| 6 | $CH_2CH=CH_2$ | H | dichlorhydrate | > 270 (d) |
| 7 | 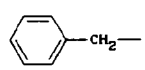 | H | dichlorhydrate | 196-199 (d) |
| 8 | Br | H | dichlorhydrate | 211-213 (d) |
| 9 | 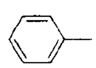 | Br | dichlorhydrate | 245-247 (d) |
| 10 | 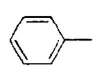 | H | dichlorhydrate | 209 (d) |
| 11 | $CH_2OCH_3$ | H | dichlorhydrate | > 270 (d) |
| 12 | $CO_2C_2H_5$ | H | dichlorhydrate | 171-174 (d) |
| 13 | $CO_2iC_3H_7$ | H | dichlorhydrate | 179-181 (d) |
| 14 | $CO_2C_2H_5$ | Cl | dichlorhydrate | 213-215 (d) |
| 15 | $CONHCH_3$ | H | dichlorhydrate | 220 (d) |
| 16 | F | H | dichlorhydrate | 203-204 |
| 17 | 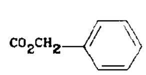 | H | dichlorhydrate | 195-199(d) |
| 18 | 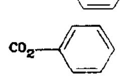 | H | dichlorhydrate | 211-214(d) |
| 19 | H | H | chlorhydrate | 212-215 |

| Composé | $R_1$ | $R_2$ | base ou sel | F (°C) |
|---|---|---|---|---|
| 20 | $CH_3$ | $CH_3$ | dichlorhydrate | 237-238 |
| 21 | Cl | Cl | dichlorhydrate | 206-213 |
| 22 | Br | Br | base | 175 (d) |
| 23 | Cl | H | dichlorhydrate | 217-219 (d) |

(d) = décomposition

Tableau II

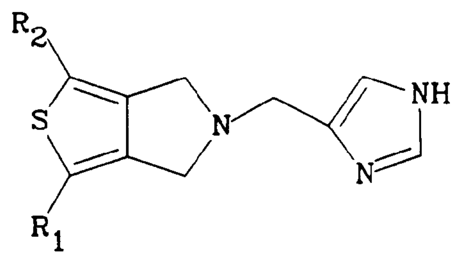

| Composé | $R_1$ | $R_2$ | Base ou sel | F(°C) |
|---|---|---|---|---|
| 24 | H | H | dichlorhydrate | 260 (d) |
| 25 | Br | Br | dichlorhydrate | 221 (d) |
| 26 | Cl | Cl | dichlorhydrate | 229-231 (d) |
| 27 | Cl | H | dichlorhydrate | 241-243 (d) |

(d) = décomposition

Les composés de l'invention présentent une activité pharmacologique $\alpha_2$-antagoniste et ont été testés dans différents essais biologiques.

1. Antagonisme des effets de la clonidine sur le vas deferens de rat.

Cette détermination a eu lieu sur le vas deferens du rat stimulé à une fréquence de 0,1 Hz en présence de 30 nanomoles de prazosine et de une micromole de cocaïne selon la méthode décrite par G.M. Drew dans European Journal of Pharmacology, 42, 123-130, (1977).
Les $pA_2$ des composés de l'invention sont compris entre 6,5 et 9,4.

2. Antagonisme de la liaison de la $^{3}H$-clonidine sur les récepteurs $\alpha_2$-adrénergiques.

Le test est réalisé sur une préparation de membranes de cerveau de rat, selon la méthode décrite par D.A. Greenberg et al. dans Life Sci. 19, 69, (1976).
Après 30 mn d'incubation en présence de clonidine tritiée (0,05 à 7 nmole/l), on filtre et on procède au comptage de la radioactivité du résidu selon la méthode de P.B.M.W.M. Timmermans et al., décrite dans European Journal of Pharmacology, 70, 7, (1981).
Les concentrations inhibitrices 50 des composés de l'invention sont comprises entre 0,02 et 3,02 µmole/l.

Les résultats des essais biologiques montrent que les composés de l'invention présentent in vitro des propriétés antagonistes vis-à-vis des récepteurs adrénergiques de type $\alpha_2$. Les composés de l'invention peuvent être utilisés, compte tenu de leurs propriétés pharmacologiques, pour le traitement du diabète, de l'obésité, de l'hypotension, de l'iléum paralytique post-opératoire et/ou de l'asthme.

Les composés de l'invention présentent également une activité $\alpha_1$-agoniste, mise en évidence par des essais biologiques sur l'artère pulmonaire isolée de lapin.

Ces essais ont été réalisés dans les conditions suivantes: des lapins (Fauve de Bourgogne), pesant 2 à 3 kg, ont été assommés et saignés, puis leurs artères pulmonaires ont été prélevées, disséquées et découpées en bandelettes d'environ 1,2 à 2 mm de large et 20 mm de long.
Ces bandelettes de tissu vasculaire ont été plongées dans une solution physiologique (composition, exprimée en mmol./l : chlorure de sodium 137 ; chlorure de potassium 2,7 ; chlorure de calcium 1,8 ; dihydrogénophosphate de sodium 0,4 ; hydrogénocarbonate de sodium 11,9 ; hexahydrate de chlorure de magnésium 1,1 ; dextrose 5,9 ; sel disodique de l'acide éthylènediaminetétraacétique 0,027 et acide ascorbique 0,057), oxygénée avec un mélange de 95% d'oxygène et de 5% de gaz carbonique et maintenue à une température de 37°C.
Elles ont ensuite été soumises, pendant 4 h, à une traction de 4 g, réduite à 2 g juste avant le début de l'expérience.
On a alors contracté le tissu avec le composé à étudier et on a enregistré la tension résultante à l'aide d'un polygraphe Grass, modèle 7D et un transducteur de force. On a tracé deux courbes concentration-effet, avec des concentrations cumulatives de composé (de 100 nmol./l à 3 mmol./l) puis on a ajouté dans le bain, un $\alpha_1$-antagoniste, l'alfuzosine, à la concentration de 1 µmol./l, laissé en contact avec le tissu pendant 30 mn. Une autre courbe concentration-effet a alors été tracée et comparée avec la seconde courbe de contrôle.

L'effet $\alpha_1$-agoniste est mesuré par la concentration provoquant une contraction égale à 50 % de l'effet maximal.

Pour les composés de l'invention, cette concentration varie entre 1,3 et 2,6 µmol./l.

Ces résultats montrent que les composés de l'invention présentent in vitro des propriétés agonistes vis-à-vis des récepteurs adrénergiques de type $\alpha_1$. Les composés de l'invention peuvent donc être utilisés dans le traitement de l'incontinence urinaire.

Les composés de l'invention peuvent être présentés sous toute forme appropriée, en association avec tout excipient approprié, pour l'administration par voie orale ou parentérale ; par exemple sous la forme de comprimés, de dragées, de capsules, de solutions, etc..;

La posologie quotidienne peut aller de 0,1 à 20 mg/kg par voie orale.

# Annexe 1

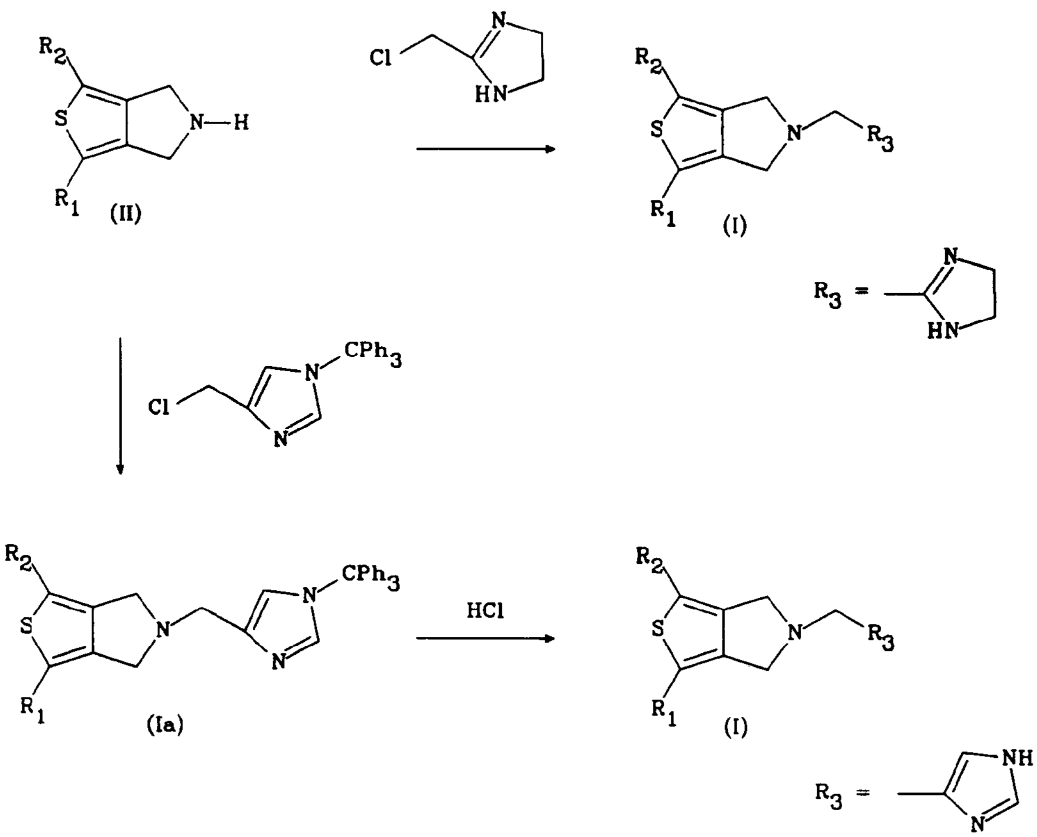


(II)

(I)

(Ia)

(I)

Annexe 2

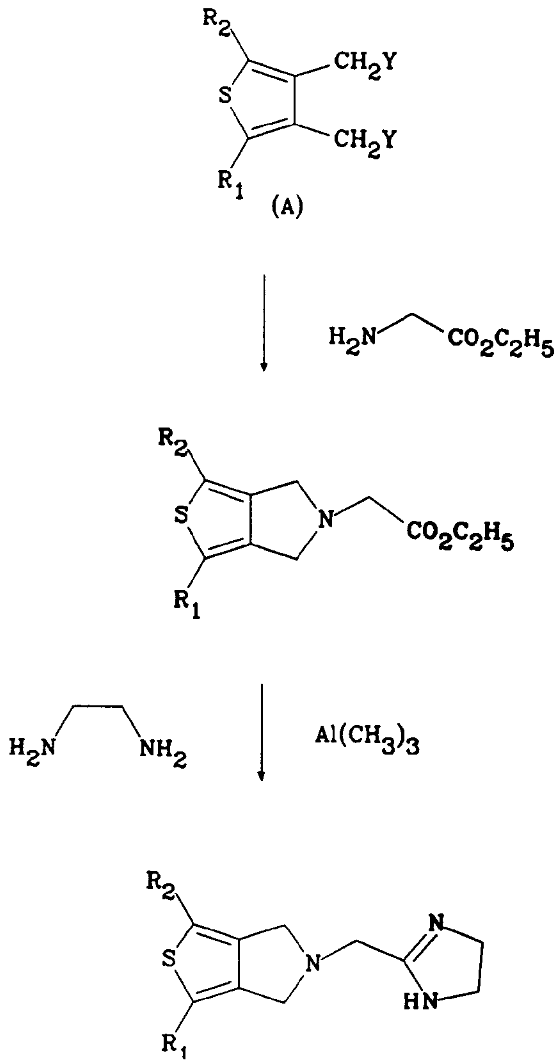

## Annexe 3

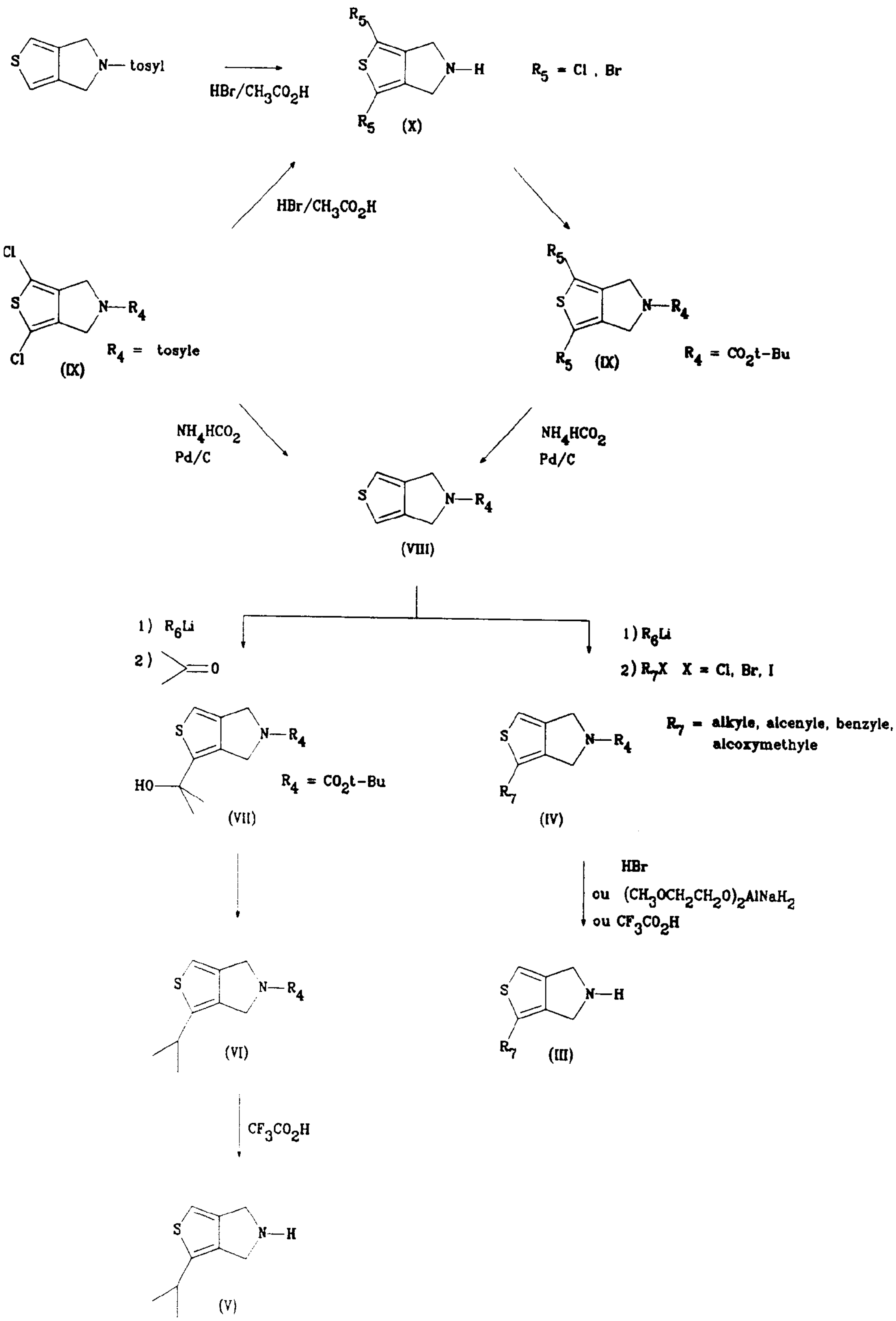
HBr/CH$_3$CO$_2$H
R$_5$ = Cl , Br
(X)
HBr/CH$_3$CO$_2$H
(IX)
R$_4$ = tosyle
(IX)
R$_4$ = CO$_2$t-Bu
NH$_4$HCO$_2$
Pd/C
NH$_4$HCO$_2$
Pd/C
(VIII)
1) R$_6$Li
2)
1) R$_6$Li
2) R$_7$X X = Cl, Br, I
R$_4$ = CO$_2$t-Bu
R$_7$ = alkyle, alcenyle, benzyle, alcoxymethyle
(VII)
(IV)
HBr
ou (CH$_3$OCH$_2$CH$_2$O)$_2$AlNaH$_2$
ou CF$_3$CO$_2$H
(VI)
(III)
CF$_3$CO$_2$H
(V)

## Annexe 4

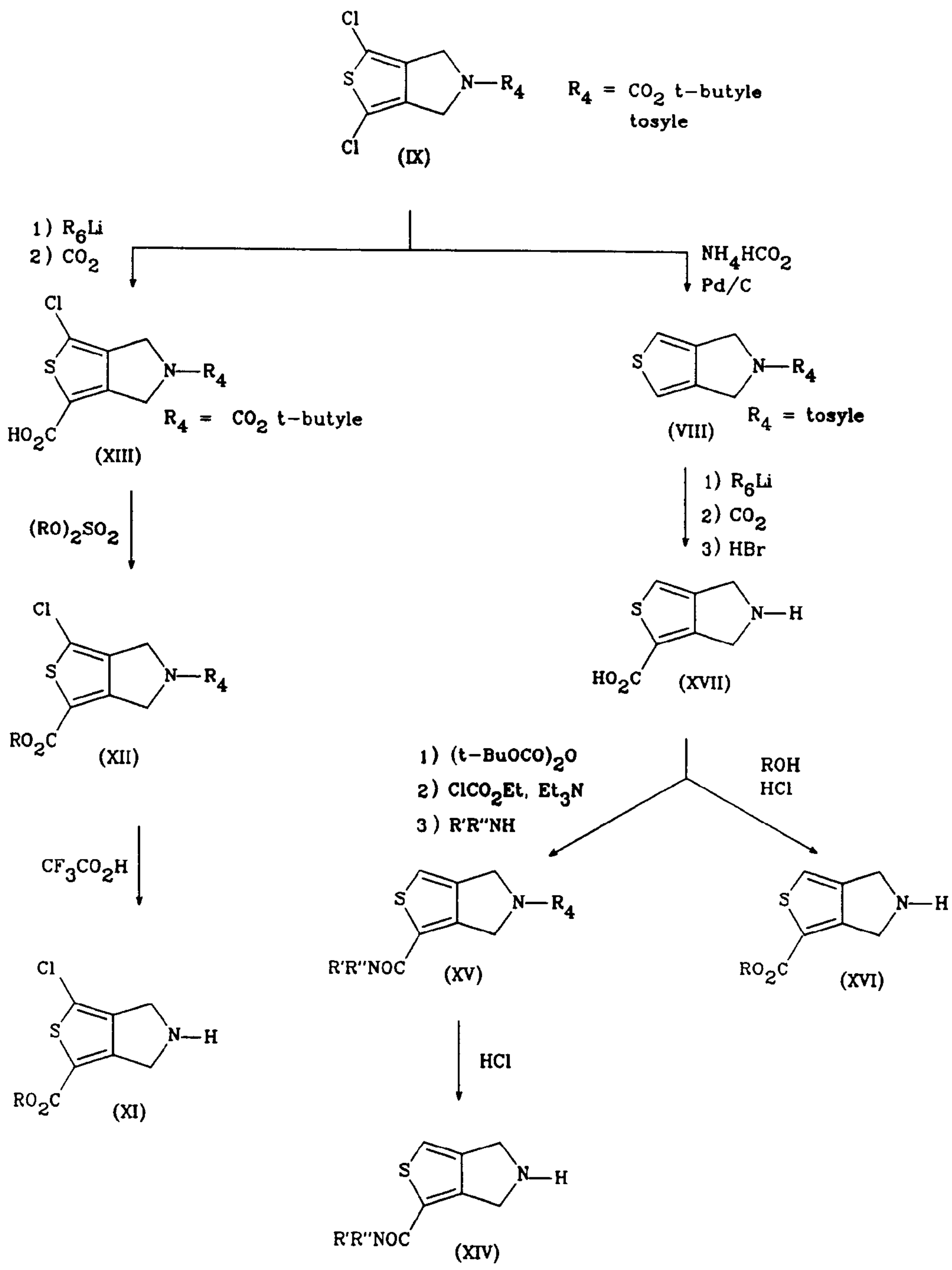
$R_4$ = $CO_2$ t-butyle
tosyle
(IX)
1) $R_6Li$
2) $CO_2$
$NH_4HCO_2$
Pd/C
$R_4$ = $CO_2$ t-butyle
(XIII)
(VIII)
$R_4$ = tosyle
1) $R_6Li$
2) $CO_2$
3) HBr
$(RO)_2SO_2$
(XVII)
(XII)
1) $(t-BuOCO)_2O$
2) $ClCO_2Et$, $Et_3N$
3) R'R"NH
ROH
HCl
$CF_3CO_2H$
(XV)
(XVI)
(XI)
HCl
(XIV)

# Annexe 5

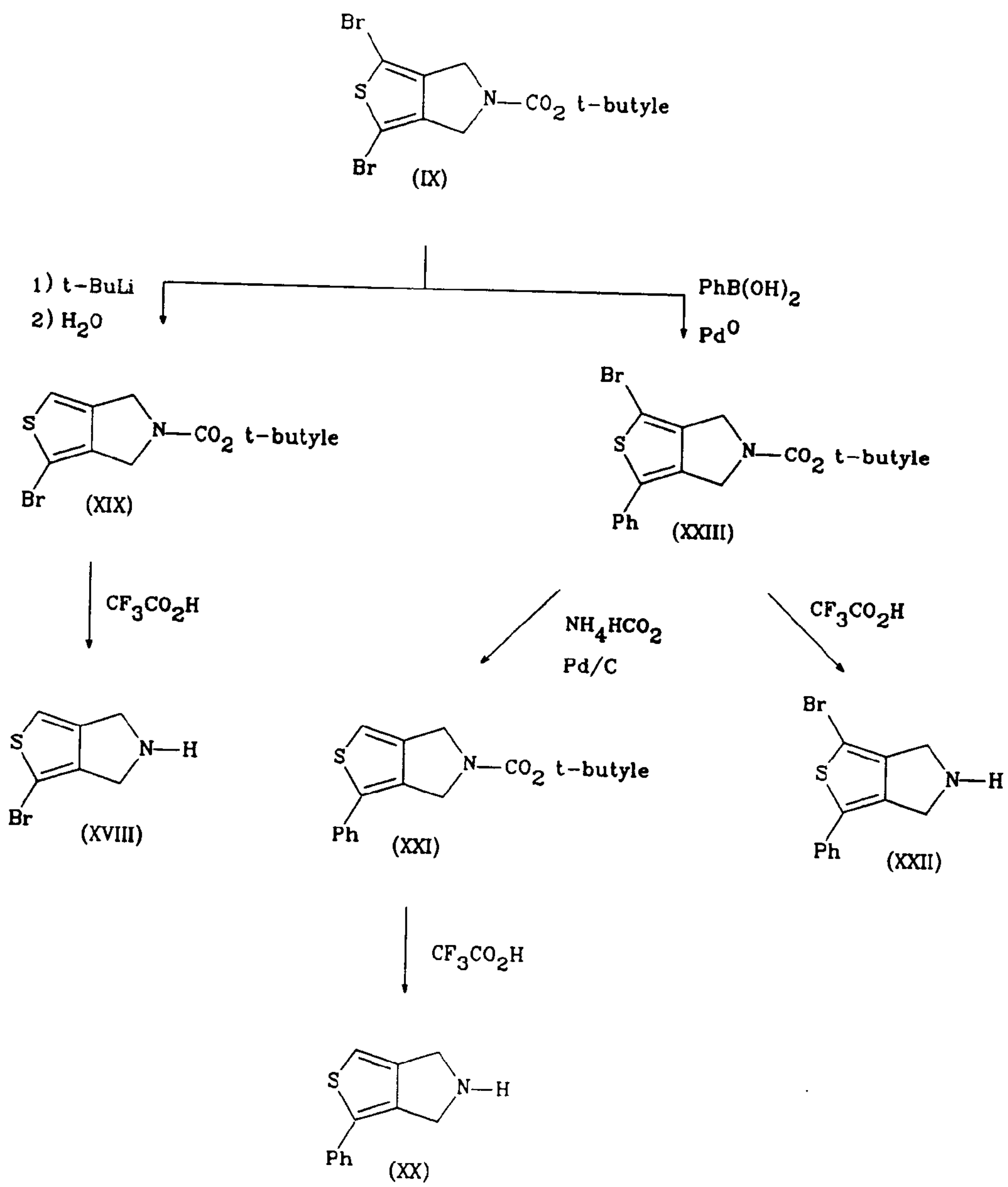
(IX)
1) t-BuLi
2) $H_2O$
$PhB(OH)_2$
$Pd^0$
(XIX)
(XXIII)
$CF_3CO_2H$
$NH_4HCO_2$
Pd/C
$CF_3CO_2H$
(XVIII)
(XXI)
(XXII)
$CF_3CO_2H$
(XX)

## Annexe 6

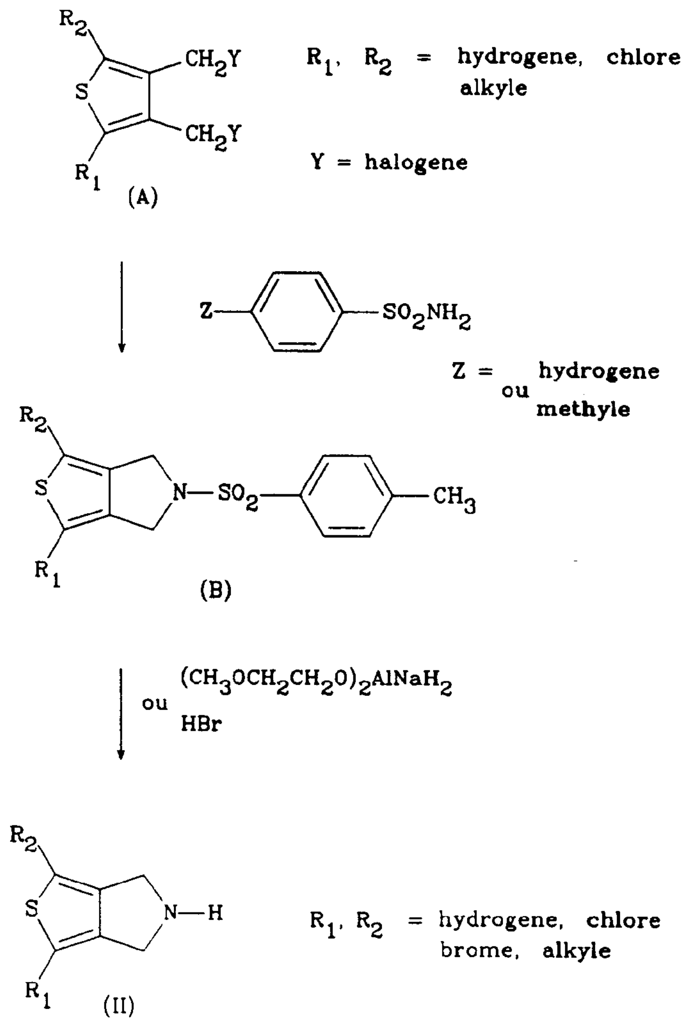


(A)

(B)

(II)

## Revendications

**1.** Dérivés de pyrrole de formule générale (I)

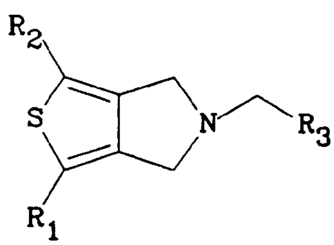

(I)

dans laquelle $R_1$ représente un atome d'hydrogène ou d'halogène, un groupe $C_{1-6}$ alkyle linéaire ou ramifié, un groupe $C_{3-6}$ alcényle, un groupe $C_{1-4}$ alcoxyméthyle, le radical benzyle, un groupe phényle éventuellement substitué par un ou plusieurs atomes d'halogène ou radicaux alkyle, un groupe $CO_2R$ dans lequel R représente un radical $C_{1-4}$ alkyle linéaire ou ramifié, le radical phényle ou le radical benzyle, ou un groupe CONR'R" dans lequel R'et R" représentent chacun, indépendamment l'un de l'autre, un atome d'hydrogène ou un radical $C_{1-4}$ alkyle linéaire ou ramifié,

$R_2$ représente un atome d'hydrogène ou d'halogène ou un groupe $C_{1-4}$ alkyle linéaire ou ramifié et $R_3$ représente un groupe 4,5-dihydro-1H-imidazol-2-yle ou 1H-imidazol-4-yle, ainsi que leurs sels d'addition à des acides pharmaceutiquement acceptables.

2. Les dérivés de pyrrole de formule générale (I), dans laquelle $R_3$ représente un groupe 4,5-dihydro-1H-imidazol-2-yle.

3. Les dérivés de pyrrole de formule générale (I), dans laquelle $R_3$ représente un groupe 1H-imidazol-4-yle.

4. Le dichlorhydrate de 1-éthyl-5-[(4,5-dihydro-1*H*-imidazol-2-yl)méthyl]-5,6-dihydro-4*H*-thiéno[3,4-*c*]pyrrole.

5. Le dichlorhydrate de 1-éthoxycarbonyl-5-[(4,5-dihydro-1*H*-imidazol-2-yl)méthyl]-5,6-dihydro-4*H*-thiéno[3,4-*c*]pyrrole.

6. Le 1,3-dibromo-5-[(4,5-dihydro-1*H*-imidazol-2-yl)méthyl]-5,6-dihydro-4*H*-thiéno[3,4-*c*]pyrrole.

7. Le dichlorhydrate de 1-chloro-5-[(4,5-dihydro-1*H*-imidazol-2-yl)méthyl]-5,6-dihydro-4*H*-thiéno[3,4-*c*]pyrrole.

8. Procédé de préparation des composés de formule (I) selon la revendication 1, dans laquelle R3 représente un groupe 4,5-dihydro-1H-imidazol-2-yle, caractérisé en ce que l'on fait réagir un composé de formule (II)

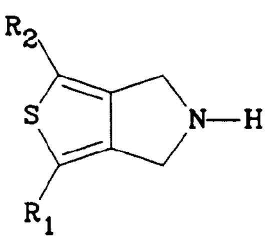

(II)

dans laquelle R1 et R2 sont définis comme dans la revendication 1, avec le 2-chlorométhyl-4,5-dihydro-1H-imidazole, dans un solvant tel que le diméthylformamide, en présence de N,N-diisopropyléthylamine, dans un bain soumis aux ultrasons.

9. Procédé de préparation des composés de formule (I) selon la revendication 1, dans laquelle R3 représente un groupe 1H-imidazol-4-yle, caractérisé en ce que l'on fait réagir un composé de formule (II) dans laquelle R1 et R2 sont définis comme dans la revendication 1, avec le 4-chlorométhyl-1-triphénylméthylimidazole, dans un solvant tel que le diméthylformamide, en présence de N,N-diisopropyléthylamine, dans un bain soumis aux ultrasons, et que l'on traite le composé obtenu, avec de l'acide chlorhydrique, à une température d'environ 100°C.

10. Médicament caractérisé en ce qu'il consiste en un composé de formule (I) selon la revendication 1.

11. Composition pharmaceutique caractérisée en ce qu'elle contient un composé de formule (I) selon la revendication 1 en association avec tout excipient approprié.

Office européen des brevets

# RAPPORT DE RECHERCHE EUROPEENNE

Numero de la demande
EP 93 40 2785

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| A | EP-A-0 238 753 (BEECHAM GROUP)<br>* revendications 1,10,13 *<br>--- | 1,11 | C07D491/048<br>C07D495/04<br>A61K31/415<br>//(C07D491/048, 307:00,209:00)<br>(C07D495/04, 333:00,209:00) |
| A | CHEMICAL ABSTRACTS, vol. 67, no. 7, 1967, Columbus, Ohio, US;<br>abstract no. 32526a,<br>K. YU. NOVITSKII ET AL 'Furan series. XL. Reaction of 3,4-bis(chloromethyl)furan with primary amines'<br>page 3070 ;<br>* abrégé *<br>& KHIM. GETEROTSIKL. SOEDIN<br>no. 6 , 1966<br>pages 818 - 821<br>--- | 1 | |
| A | JOURNAL OF ORGANIC CHEMISTRY.<br>vol. 34, no. 2 , 1969 , EASTON US<br>pages 333 - 340<br>D. J. ZWANENBURG ET AL 'Steric inhibition of intramolecular cyclizations by ortho substituents. The synthesis of 1H,3H-thieno(3,4-c)thiophene, its 2,2-dioxide, and 5-ethyl-5,6-dihydro-4H-thieno(3,4-c)pyrrole'<br>* page 336, composé 28 *<br>--- | 1 | DOMAINES TECHNIQUES RECHERCHES (Int.Cl.5)<br>C07D<br>A61K |
| P,A | WO-A-93 03714 (THE UPJOHN COMPANY)<br>* revendication 1; exemples 95,104 *<br>----- | 1,11 | |

Le présent rapport a été établi pour toutes les revendications

| Lieu de la recherche | Date d'achèvement de la recherche | Examinateur |
|---|---|---|
| LA HAYE | 14 Février 1994 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)